# EUROPEAN PATENT SPECIFICATION

(11) **EP 3 569 149 B1**
(45) Date of publication and mention of the grant of the patent: **07.08.2024**
(21) Application number: 18739311.1
(22) Date of filing: 10.01.2018
(51) Int. Cl.: A61B 6/51

(54) **BONE DENSITY MEASUREMENT DEVICE, BONE DENSITY MEASUREMENT SYSTEM, AND IMAGING ASSISTING TOOL**
VORRICHTUNG ZUR MESSUNG DER KNOCHENDICHTE, KNOCHENDICHTEMESSSYSTEM UND BILDGEBUNGSASSISTENZINSTRUMENT
DISPOSITIF DE MESURE DE DENSITÉ OSSEUSE, SYSTÈME DE MESURE DE DENSITÉ OSSEUSE ET OUTIL D'AIDE À L'IMAGERIE

(30) Priority: 10.01.2017 JP 2017002038
(43) Date of publication of application: 20.11.2019
(73) Proprietor: Media Co., Ltd., Bunkyou-ku, Tokyo 113-0033 (JP)
(72) Inventor: SHIMODA, Shinji, Yokohama-shi Kanagawa 230-8501 (JP); KOBAYASHI, Kaoru, Yokohama-shi Kanagawa 230-8501 (JP); KATSUMATA, Akitoshi, Mizuho-shi Gifu 501-0296 (JP); TSUJI, Hironobu, Tokyo 113-0033 (JP); TSUJI, Yosuke, Tokyo 113-0033 (JP); HAYASHI, Tatsuro, Tokyo 113-0033 (JP)
(74) Representative: Schön, Christoph
(86) International application number: PCT/JP2018/000357
(87) International publication number: WO 2018/131611

(56) References cited:
- JP-A- 2001 120 530
- JP-A- 2004 174 162
- JP-A- 2004 174 162
- JP-A- 2004 275 334
- JP-A- 2007 136 213
- JP-A- 2016 523 162
- JP-A- H06 285 059
- JP-A- H06 285 059
- JP-A- S62 266 053
- JP-A- S62 266 053
- JP-B2- 3 300 690
- JP-B2- 4 077 430
- JP-B2- H07 102 210
- US-A1- 2002 067 798
- US-A1- 2007 025 607
- US-B1- 6 320 931
- US-B1- 8 186 880
- SOUTHARD T E ET AL: "Geometric and densitometric standardization of intraoral radiography through use of a modified XCP system", ORAL SURGERY, ORAL MEDICINE, ORAL PATHOLOGY, ORAL RADIOLOGY AND ENDODONTICS, MOSBY-YEAR BOOK, ST. LOUIS, MO, US, vol. 87, no. 2, 1 February 1999 (1999-02-01), pages 253 - 257, XP027500734, ISSN: 1079-2104, [retrieved on 19990201], DOI: 10.1016/S1079-2104(99)70281-7
- YANG ET AL: "A new device for measuring density of jaw bones", vol. 31, no. 5, 1 September 2002 (2002-09-01), pages 313 - 316, XP009531389, ISSN: 0250-832X, Retrieved from the Internet <URL:https://doi.org/10.1038/sj.dmfr.4600715?locatt=mode:legacy> [retrieved on 20140128], DOI: 10.1038/SJ.DMFR.4600715

## Description

### Technical field

The present invention relates to an apparatus and system for measuring bone density using an X-ray image of a bone, and in particular, a bone density measuring apparatus for quantitatively calculating the bone density of alveolar bone from dental X-ray images, and The present invention relates to a bone density measurement system. It also relates to an imaging aid for making the measurement.

### Background technology

In the field of dental treatment, X-ray imaging is widely performed at the time of treatment. In recent years, the bone density of alveolar bone has been measured from the photographed image, and it is useful for treatment.

For example, Patent Document 1 measures the density pattern of alveolar bone by measuring the shading density of the X-ray image of alveolar bone using an aluminum standard substance, and then the alveolar bone width, absorption area and maximum absorption from the density pattern A technical idea of a method for evaluating the degree of bone atrophy of alveolar bone characterized in that at least one index of degree is determined and the degree of bone atrophy of alveolar bone is evaluated by the index is disclosed.

Here, as a quantitative index of the degree of bone atrophy, indices such as alveolar bone width, resorbed area and maximum absorbency are used.

Further, Patent Document 2 shows a bone density evaluation apparatus for evaluating bone density based on a radiograph image of a mandible, in particular, an alveolar bone part around a first premolar tooth, and the radiography image is aligned with the mandible Detection means for detecting the lightness / darkness of the image of the sample body including the image of the sample body (aluminum block) arranged in step b), and the lightness / darkness of the X-ray image taken so that the detection result by the detection means matches the reference value. A technical idea of a bone density evaluation apparatus is disclosed, characterized by comprising a correction means for correction and an evaluation means for evaluating a bone density based on the corrected degree of density corrected by the correction means.

Here, for example, with respect to a plurality of image data captured on different days of the same patient, it is possible to quantitatively evaluate the bone density regardless of the difference in lightness and darkness of each X-ray image.

Further, in Patent Document 3, the density of the object to be measured, such as teeth and bones of the human body, and the amount of inorganic salt can be quantitatively calculated. Disclosed is a standard sample having a composition and compactness and containing no impurities, and a technical idea of a method for producing the same.

According to this, when quantitatively evaluating a bone density from a radiographic image, the composition desirable as a standard sample and its manufacturing method are shown.

### Prior Art Literature

### [Patent Document]

[Patent Document 1] Japanese Patent Application Laid-Open No. 62-266053
[Patent Document 2] Japanese Patent No. 4077430
[Patent Document 3] Patent No. 3300690
Article SOUTHARD T E ET AL: "Geometric and densitometric standardization of intraoral radiography through use of a modified XCP system",ORAL SURGERY, ORAL MEDICINE, ORAL PATHOLOGY, ORAL RADIOLOGY AND ENDODONTICS, MOSBY-YEAR BOOK, ST. LOUIS, MO, US, vol. 87, no. 2, 1 February 1999 (1999-02-01), pages 253-257 describes an imaging aid for x-ray dental densitometry, so that a reference stepwedge is imaged together with the tooth. It further suggests approximation of the a relation between an X-ray image density of the plurality of stepwedge steps and the corresponding bone density values via piecewise linear, third-degree polynomial, or fourth-degree polynomial curves.

### SUMMARY OF THE INVENTION

### [Problems to be solved by the invention]

However, in the bone atrophy degree evaluation method of Patent Document 1, the bone mass per unit area (g / square cm), which is a general expression method of bone density, can not be expressed, and therefore evaluation with universality There was a problem that it was hard to say the result.

Also, in the bone density evaluation apparatus of Patent Document 2, although the density degree for each image can be corrected and compared quantitatively, it can be expressed by a general bone density expression method as well. There was a problem of not being.

In addition, about the standard sample of patent document 3, in view of the effectiveness, it is desirable to aim at the application positively. In the present invention, a standard sample is expressed as a reference.

In addition, none of the patent documents disclose effective imaging aids for imaging the reference object and the measurement object, that is, the bone portion, particularly the alveolar bone portion, on the same screen.

Therefore, in order to solve the above-mentioned problems, the present invention accurately measures the apparatus and system for quantitatively calculating the bone density using the X-ray image of the photographed bone part, particularly the alveolar bone, and the X-ray imaging of the alveolar bone correctly It was an object of the present invention to provide an imaging aid for easily and easily. The invention is defined in the appended claims.

### [Means for Solving the Problems]

In order to solve such problems, the present invention relates to a bone density measuring device, An image display unit for displaying an X-ray image relating to a photographed bone part and a reference body on the same screen; A reference body concentration measuring unit that measures the density of the X-ray image of the displayed reference body;
- A bone density measurement unit for measuring the density of the displayed X-ray image of the bone;
- A bone density calculation unit for calculating the bone density of bone parts by comparing the X-ray image density for bone parts with the X-ray image density for reference bodies It is characterized by having.

In this way, the reference body concentration measuring unit and the bone portion concentration measuring unit measure the concentrations (pixel values or gradations of the gray scale image) between the reference body and the bone portion region of interest, and then calculate the bone density. By comparing these concentrations by the department, the bone density can be calculated as an absolute value, and accurate and universal bone density evaluation results can be obtained.

Further, the bone density measuring device of the present invention may be characterized in that the reference body has a composition close to that of the teeth.

In this way, the correlation of the image density between the reference body and the bone portion can be obtained more accurately, and the bone density value with high accuracy can be obtained.

Furthermore, in the bone densitometry device of the present invention, the reference body is constituted by mixing hydroxyapatite or a hydroxyapatite homolog in which the OH group of the apatite is substituted with another element and carbon in different mixing ratios It may be characterized by

In this way, in particular the most suitable reference is obtained and the correlation is further accurate.

Furthermore, the bone densitometry device of the present invention may be characterized in that the reference body includes samples from which three levels of image density can be obtained, and the bone density is calculated using them.

In this case, the number of stages of the reference body is reduced, so that the processing in the imaging stage, the comparison stage, and the bone density calculation stage becomes simple, while in the present invention, the practical accuracy is sufficient even with three stages. Economic benefits are great because it can be maintained.

Furthermore, the bone densitometry device of the present invention may be further characterized in that detection of the reference body is started by reading a marker attached to the case containing the reference body.

In this way, using the markers simplifies the device and detection procedure and provides accurate data.

Furthermore, the method may be characterized by including a reference body region of interest setting unit that sets a region of interest of the reference body after detection of the reference body is started.

In this way, even when the density of the reference body is not uniform, many pixels can be sampled to obtain a representative value (average value, median value, mode value, etc.), so more accurate detection is possible. It becomes.

Furthermore, according to another aspect of the present invention, there is provided a bone densitometry system comprising: a reference body for bone densitometry; an imaging device for simultaneously taking an X-ray image of a bone portion and the reference body; And a density measuring device.

In this way, as a system including the bone densitometry device, the effect of the bone densitometry device can be sufficiently exhibited.

In addition, the reference body of the present invention may be characterized by having a composition close to that of a tooth. In this way, as a system including the reference body and the bone densitometry device, the effect of the bone densitometry device can be sufficiently exhibited.

Furthermore, in the bone density measurement system of the present invention, the reference body is constituted by mixing hydroxyapatite or a hydroxyapatite homolog in which the OH group of the apatite is substituted with another element and carbon in different mixing ratios It may be characterized by Also as a system including the reference body and the bone densitometry device, the effect of the bone densitometry device can be sufficiently exhibited.

The bone densitometry system of the present invention may also be characterized in that the reference includes a sample from which three levels of image density can be obtained. According to this, as a system including the reference body and the bone densitometry device, the effect of the bone densitometry device can be sufficiently exhibited.

Furthermore, the bone density measurement system of the present invention may be characterized by having a case for housing a reference body and an automatically readable marker provided on the case. In this way, by using the marker as a system including the reference body and the bone densitometry device, the effect of the bone densitometry device can be sufficiently exhibited.

Furthermore, the bone densitometry system of the present invention is characterized by having an imaging aid for holding the reference body for simultaneously imaging the reference body and the bone portion.

In this way, X-ray imaging of the reference body and alveolar bone can be performed accurately and easily, and the burden on the subject can be reduced, and highly accurate bone density measurement results can be obtained.

Further, according to the present invention, there is provided a photographing aid, comprising: a holding unit for holding an image information detection medium for photographing a bone portion; and one end of the holding unit to support the holding unit in an appropriate position. The support portion may be provided with a mechanism for holding the reference body so that the image information detection medium is detected together with the bone portion on the support portion side of the holding portion.

In this way, as a photographing aid for holding a reference body simultaneously photographed with the bone for measuring bone density, not only the bone density measuring system of the present invention but also other bone density measuring systems are used. Is also effective.

In the bone density measuring device and the bone density measuring system according to the present invention, the image comparing unit compares the reference body with the region of interest by using an appropriate reference body, and the bone density calculating unit further calculates the bone density in absolute value. Because it is calculated, accurate and universal bone density values can be obtained.

### Brief Description of the Drawings

FIG. 1 is a hardware configuration diagram of a bone density measurement system according to an embodiment of the present invention.
FIG. 2 is an illustration of a reference body of a system according to an embodiment of the present invention.
FIG. 3 is an explanatory view of a photographing aid of a system according to an embodiment of the present invention.
FIG. 4 is an illustration of another imaging aid of the system according to an embodiment of the present invention.
FIG. 5 is a functional configuration diagram of a bone density measurement device according to an embodiment of the present invention.
FIG. 6 is an operation flow of a system according to an embodiment of the present invention.
FIG. 7 is an example of a captured image of a system according to an embodiment of the present invention.
FIG. 8 is an operation flow of setting a reference body region of interest of a system according to an embodiment of the present invention.
FIG. 9 is an example of an image of a reference body region of interest setting unit of the system according to the embodiment of the present invention.
FIG. 10 is an example of an image of a reference body region of interest setting unit of the system according to the embodiment of the present invention.
FIG. 11 is a correlation diagram of image density and bone density of a system according to the present invention.
FIG. 12 is an explanatory view of a method of setting a bone part region of interest of the device according to one embodiment of the present invention;
FIG. 13A is an explanatory view showing an example of a bone part region of interest of the device according to the embodiment of the present invention.
FIG. 13B is an explanatory view showing an example of a bone part region of interest of the device according to one embodiment of the present invention.
FIG. 13C is an explanatory drawing showing an example of a bone part region of interest of the device according to one embodiment of the present invention.
FIG. 13D is an explanatory view showing an example of a bone portion region of interest of the device according to the embodiment of the present invention.
FIG. 13E is an explanatory view showing an example of a bone portion region of interest of the device according to one embodiment of the present invention.
FIG. 13F is an explanatory drawing showing an example of a bone part region of interest of the device according to one embodiment of the present invention.
FIG. 14 is another example of a captured image of a system according to an embodiment of the present invention.

### MODE FOR CARRYING OUT THE INVENTION

Hereinafter, a bone density measurement system and a bone density measurement apparatus according to a first embodiment of the present invention will be described with reference to the drawings. In the following, the range necessary for the description of achieving the object of the present invention is schematically shown, the range necessary for the description of the corresponding part of the present invention is mainly described, and the description is omitted. It shall be based on a well-known technique.

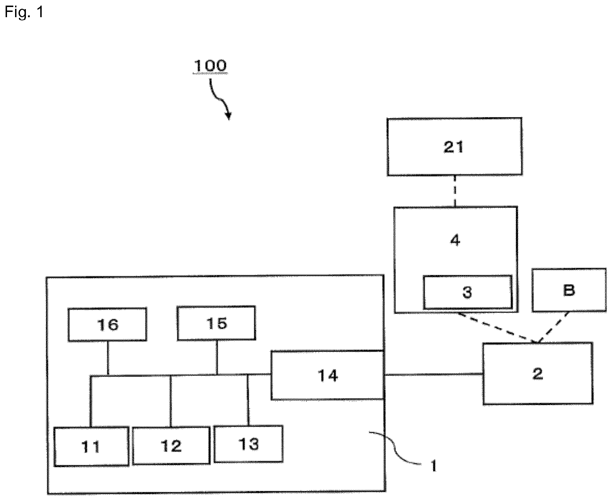

FIG. 1 is a hardware configuration diagram of a bone density measurement system 100 according to an embodiment of the present invention. The bone density measurement system 100 includes a bone density measurement device 1, an imaging device 2, a reference body 3, and an imaging aid 4. Note that the photographing aid 4 may not be used depending on the situation, or different photographing aids may be used.

Here, the bone density measuring apparatus 1 stores a display unit 11 for displaying character information and image information, an input unit 12 such as a keyboard for inputting information, an output unit 13 such as a printer for outputting information, and information and programs. Storage unit 14 such as a hard disk, an interface unit 15 connected to the outside by wire and / or wirelessly, a control unit 16 such as a CPU for controlling the whole, and so on, which may be a personal computer, a server, etc. It may be a machine. Also, these components may be integrally configured, or distributed as in cloud computing.

The imaging device 2 is an imaging device that performs radiography and the like of a dental alveolar bone portion. Although a dental X-ray imaging apparatus is suitable, it is not limited thereto, and a dental panoramic X-ray imaging apparatus, an X-ray imaging apparatus not limited to dentistry, an imaging apparatus by MRI, CT, etc., an imaging apparatus by ultrasonic waves Or a combination thereof. Depending on the images obtained, it may be possible to make an appropriate bone density measurement, however modalities except X-ray do not fall within the scope of the invention.

The imaging apparatus 2 includes an image information detection medium provided apart from the apparatus main body. As the image information detection medium, for example, an imaging plate 21 such as a plate in which a photostimulable phosphor powder is coated on one side of an organic film is suitable, but it is not limited thereto. A solid semiconductor detector such as a film (X-ray film) or a flat panel detector (FPD) may be used as the image information detection medium.

The reference body 3 is disposed so as to be reflected on the same screen when photographing a bone portion of the subject, in particular, an alveolar bone portion (outside of the configuration of the present invention). Here, it is preferable that the reference body 3 has a composition close to that of the tooth, and more specifically, hydroxyapatite or a homolog of hydroxyapatite obtained by replacing the OH group of the apatite with another element, and carbon. More preferably, they are mixed at different mixing ratios.

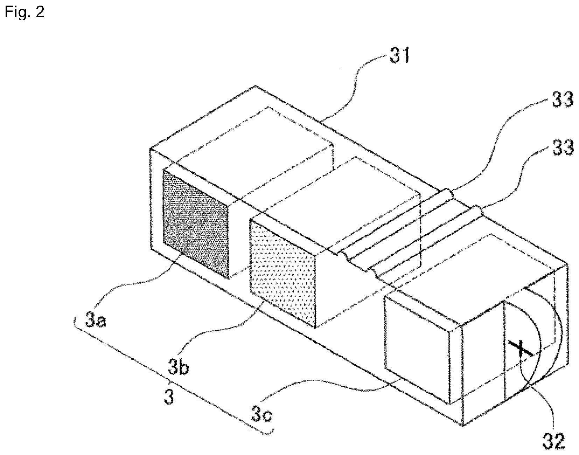

FIG. 2 is an explanatory view of a portion related to the reference body 3 of the bone density measurement system 100 according to an embodiment of the present invention. The reference 3 is composed of samples 3a, 3b and 3c from which three levels of image density can be obtained. Here, the correlation between each sample of the reference body 3 and the bone density equivalent value is, for example,

Reference body 3a mixing ratio 20% bone density equivalent value 0.50 g / square cm

Reference body 3b mixture ratio 60% bone density equivalent value 0.75 g / square cm

Reference body 3c Mixing ratio 100% Bone density equivalent value 1.00 g / square cm

It is manufactured to be. Here, the mixing ratio is the ratio of homologues of hydroxyapatite to the whole, also referred to as apatite equivalent. Practical accuracy is obtained with a three-step reference. Of course, more detailed stages, for example, four to five stages of references may be used, which makes handling more complicated, but can be expected to improve the accuracy.

Although the determination of the equivalent bone density value of the reference body may be a theoretical value calculated from the composition of the reference body, the correlation between the concentration and the bone density has been determined, for example, gbone mineral quantitative phantom h or gbone density You may obtain | require by imaging a reference body side by side with the sample called a standard chart.

The approximate dimensions of the reference body 3 are, for example, a square having a side of 5 mm on a side facing the photographing device 2 and a depth of about 10 mm, but the dimensions are limited to this size. Since it is customary that bone density is expressed in g / square cm, the thickness may be similar to that of a hard tissue such as a bone to be measured. For example, it may be 8 mm for the lower front teeth, 10 mm for the premolars, and 12 mm for the molars.

Further, the reference body 3 is inserted into and accommodated in the hole provided in the case 31, and the case 31 further extends in the direction of the reference body 3 by, for example, a copper metal wire or the like on the extension of the row of reference bodies. There is an automatically readable marker 32 formed in the form of a short cross in the direction perpendicular thereto. The position, shape, etc. of the marker are not limited to those shown in this figure, as long as automatic reading is easy, and the insertion direction of the reference body 3 is not limited to the side from the marker 32 as shown. , From the opposite side, from the top, or from the bottom.

Furthermore, two protrusions 33 are provided on the top surface of the case 31. This is for positioning and fixing at the time of assembly.

The approximate dimensions of the case 31 are preferably, for example, about 7 mm ~ 24 mm on the side facing the imaging device 2 and about 10 mm in depth, but are not limited thereto. It is not always necessary to be accommodated in the case 31. Automatic reading may be performed using the position of the image, the shape of the reference body, etc. without the marker 32, or the operator of the apparatus Reading may be supported by an input unit such as a mouse.

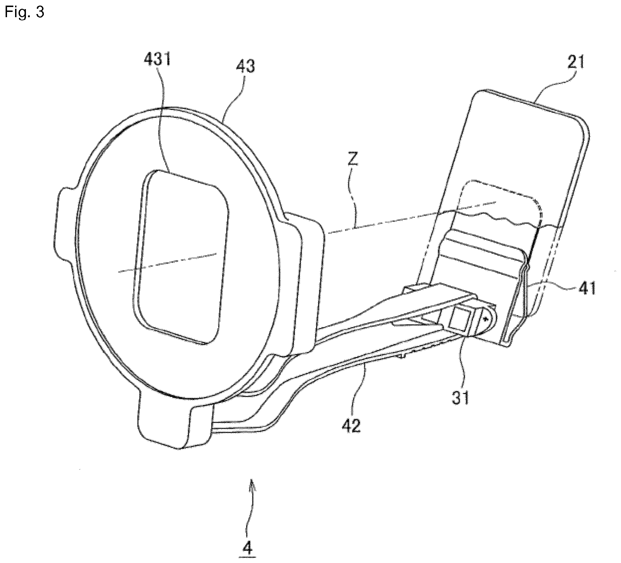

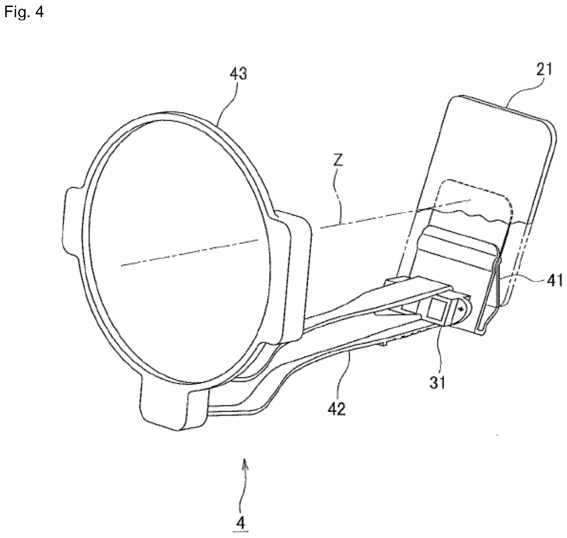

FIG. 3 and FIG. 4 are explanatory diagrams of the imaging aid 4 of the bone density measurement system 100 according to the embodiment of the present invention. Here, the photographing aid 4 holds the reference body 3 in order to simultaneously photograph the reference body 3 and the alveolar bone portion B, and the reference body 3 is accommodated in the case 31 to which the marker 32 is attached. It shall be held in the state. However, it is not necessary to be housed in the case 31 and the case where the case 31 is not used, for example, the reference body 3 itself may be fixed to the photographing aid 4 and held.

The imaging aid 4 holds the planar imaging plate 21 for capturing an X-ray image at one end of the holding portion 41 and the holding portion 41 sandwiching and holding the planar imaging plate 21 substantially orthogonal to the imaging main axis Z of the main body of the imaging device 2 An elongated rod-like support portion 42 is provided to be substantially orthogonal to 41 and is held on the opposite side of the holding portion 41 of the support portion 42 for supporting the holding portion 41 in an appropriate position by engaging the upper and lower teeth with the upper and lower teeth. In particular, in FIG. 3, it is a space provided in the center of the direction indication guide 43 so that the photographed image is rectangular. To have a rectangular stop 431.

Note that FIG. 4 does not have the rectangular stop 431 and the inside of the direction indication guide 43 is a large space, and such a simple structure may be used if it is not necessary to narrow it to the rectangular shape. Good. Furthermore, the direction indication guide 43 itself may not necessarily be provided depending on the photographing method and the like.

Furthermore, at the end of the support portion 42 on the holding portion 41 side, the case 31 accommodating the reference body 3 is installed so as to be detected by the imaging plate 21 together with the alveolar bone portion.

Specifically, the case 31 containing the reference body 3 is inserted into a substantially U-shaped space formed at the end of the support portion 42 on the side of the holding portion 41, and two places provided on the case 31 side The projection 33 is fixed by fitting in a groove on the side of the support 42 not shown.

Here, the installation place and the fixing method of the case 31 accommodating the reference body 3 are not limited to the above description, and may be installed in the holding portion 41, and the fixing method may be a known method such as screwing. Alternatively, the reference body 3 and the marker 32 may be fixed to the support portion 42 or the holding portion 41 without using the case 31.

The imaging aid 4 may be used for imaging bone parts other than alveolar bone parts. In that case, the shape of the holder 4 for holding the imaging plate 21 and the case 31 containing the reference 3 or the reference 3 itself and the support 42 for properly supporting the holder 41 is It should be designed together, in short, it should be able to capture the bone and the reference body 3 on the same screen.

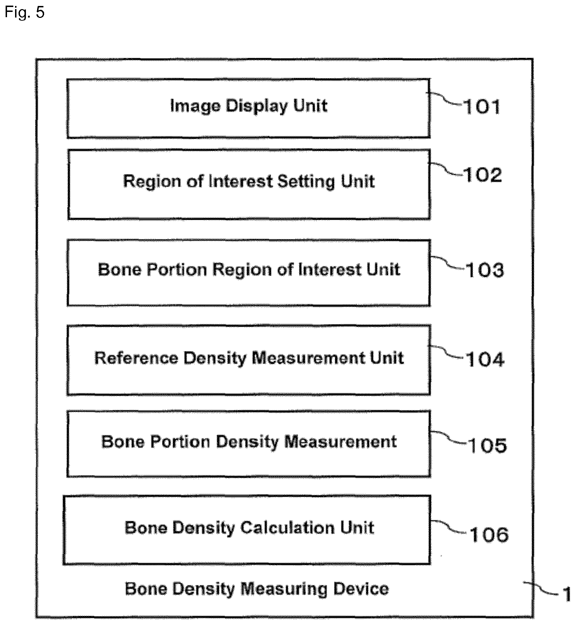

FIG. 5 is a functional configuration diagram of a bone density measurement device 1 according to an embodiment of the present invention. The bone density measuring apparatus 1 has the same screen of the X-ray images of the bones such as alveolar bones and the reference body for bone density measurement taken as the functional elements realized by the hardware components described above in cooperation with each other. The image display unit 101 displayed on the screen, the reference body region of interest setting unit 102 for setting the region of interest of the displayed reference body image, the reference body concentration measurement unit 103 for measuring the concentration of the set reference body region of interest, displayed A bone region of interest setting unit 104 for setting a region of interest in an image of a bone portion such as alveolar bone, a bone portion concentration measurement unit 105 for measuring the concentration of bone region of interest, an image density of bone region of interest and an image of a reference body It has a bone density calculation unit 106 or the like that calculates the bone density of the bone region of interest by comparing the concentration with the concentration.

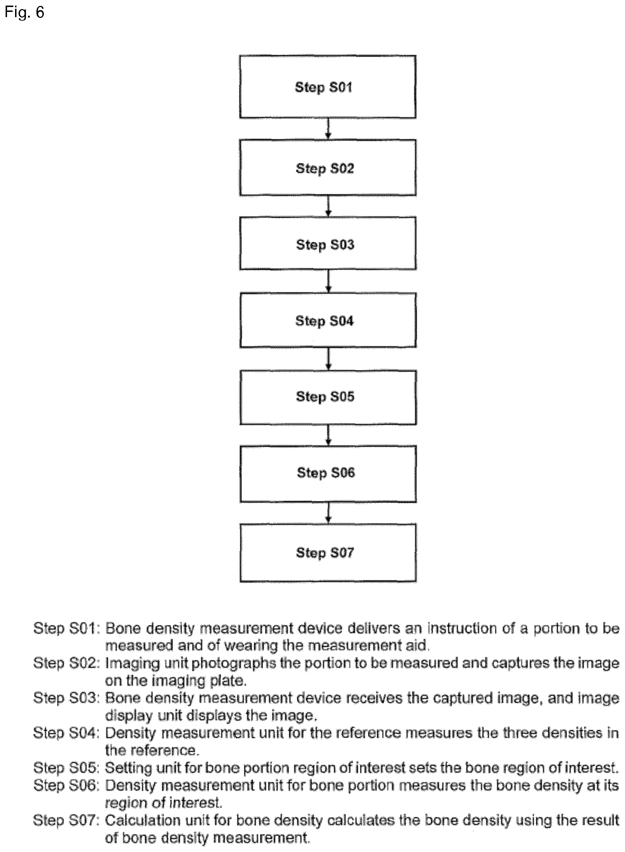

The operation of the bone density measurement system and the bone density measurement unit of these configurations will be described. FIG. 6 is an operation flow of a bone density measurement system 100 according to an embodiment of the present invention. The bone density measuring apparatus 1 indicates the region to be measured for the subject whose bone density is to be measured via the display unit 11, and instructs attachment of the imaging aid 4 (step S01). Specifically, in the case of the maxillary teeth, the tip portion (tooth top portion) of the tooth to be imaged is placed on the case 31 storing the reference body 3 and the holding portion 41 holding the imaging plate 21 is targeted The occlusal portion 42 is engaged with the subject and fixed so as to be directed to the root direction (upward) inside the teeth. In the lower and lower teeth, the upper and lower sides of the holding portion 41 are reversed.

Next, the imaging device 2 is fixed with its leading portion abutting on the direction indication guide 43 so that the imaging main axis Z is substantially perpendicular to the imaging plate 21 so that the reference body 3 and the alveolar bone portion are the same The image is taken by the intraoral method so that the image is captured on the imaging plate 21 (step S02).

Next, the bone densitometry device 1 is read from the imaging plate 21 via the main body of the imaging device 2 or by causing the imaging plate 21 to be read directly by the input unit 12 of the bone densitometry device measurement device 1 The photographed image information is received, and the image display unit 101 displays the photographed image (step S03).

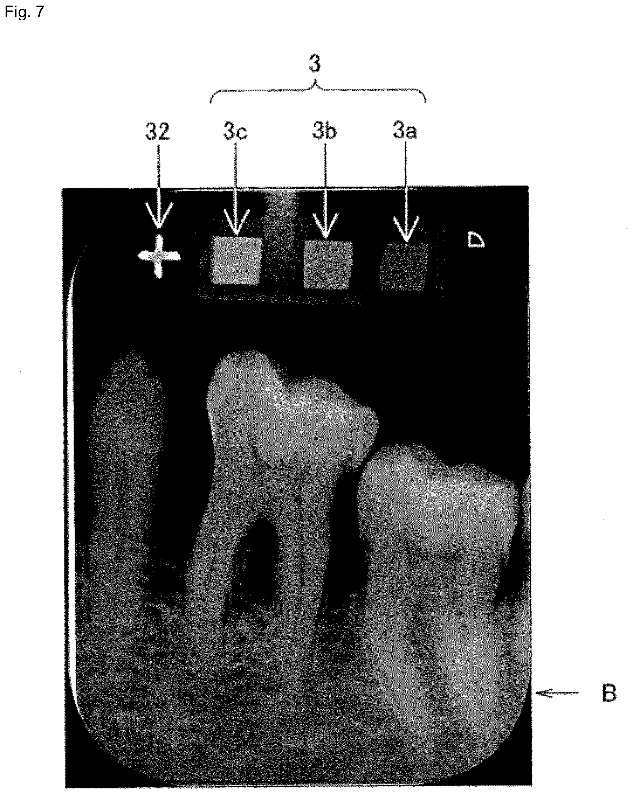

FIG. 7 shows an example of a photographed image of the bone density measurement system according to an embodiment of the present invention. The image display unit 101 of the bone density measurement device 1 displays the image of the alveolar bone portion B in the lower part. The image of is placed at the top and displayed on the same screen. Here, in the image of the reference body 3, the images of the reference body samples 3a, 3b and 3c from which the image density of three levels can be obtained sequentially from the left in the figure and the image of the marker 32 for automatic measurement are displayed. Be done.

In the drawing, the marker 32 and the reference body 3 are not aligned in the horizontal direction, and are photographed in a slightly inclined state. There are various situations of shooting, and even when they shift from the horizontal direction as described above, it often occurs, so a method that can accurately grasp the reference body 3 is required.

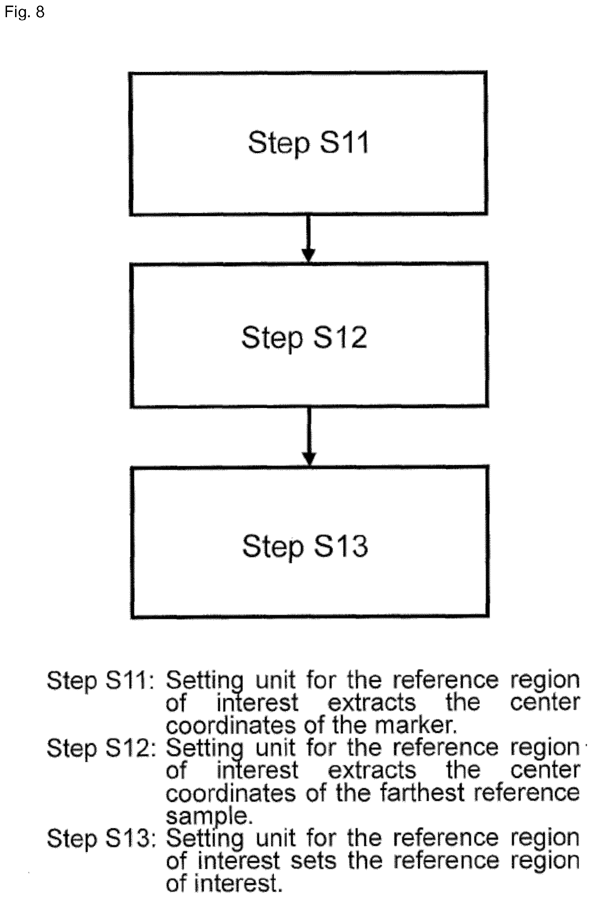

For that purpose, the reference body region of interest setting unit 102 sets a region of interest for the reference body. FIG. 8 is an operation flow of setting a reference body region of interest of the bone density measurement system according to an embodiment of the present invention.

First, center coordinates of the marker 32 are extracted on the image (step S11). Specifically, using the fact that the image of the marker 32 is a relatively thin line and having high luminance, only the image of the marker 32 part is determined by a method such as gray scale OPENING processing, image difference processing, etc., and its center coordinates Extract (Position) In addition, extraction of the center coordinates of the marker 32 is not limited to this method, and may be searched by pattern matching, for example.

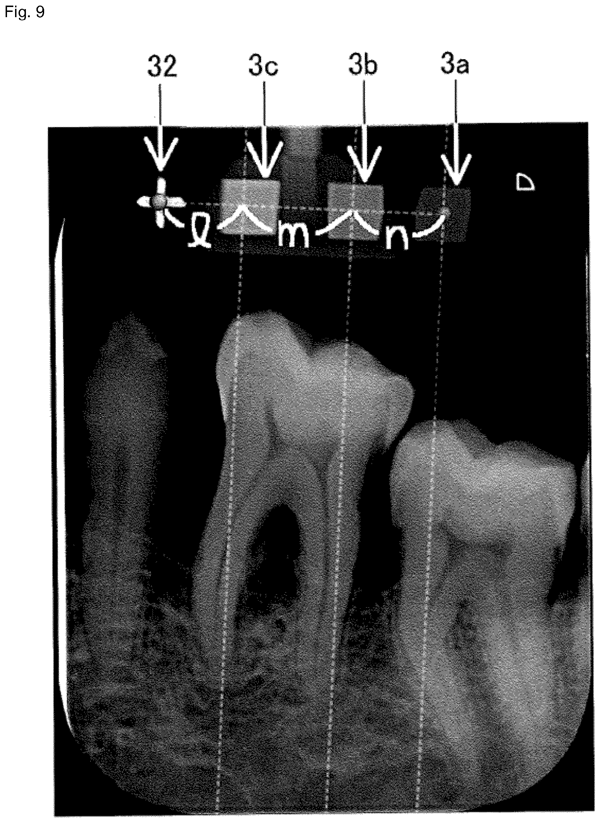

Next, using the center coordinates of the marker 32, the center coordinates of the farthest reference body sample 3a are determined (step S12). Specifically, the side in the substantially horizontal direction of the marker 32 is extended, and the position of the distance on the image corresponding to the distance to the farthest reference body sample 3c set in advance is set as the center coordinate. FIG. 9 is an example of an image related to the reference body region of interest setting unit of the bone density measurement system according to one embodiment of the present invention, in which the center coordinates of the marker 32 and the center coordinates of the farthest reference body sample 3a are shown.

Next, the region of interest of the reference body is determined using the center coordinates of the marker 32 and the center coordinates of the farthest reference body sample 3a (step S13). Specifically, as shown in FIG. 9, the distance between the center coordinates of the marker 32 and the center coordinates of the farthest reference body sample 3a is divided by a predetermined ratio (for example, as shown in FIG. 9). : M: n) Then, the center coordinates of the other reference body samples 3c, 3b are determined.

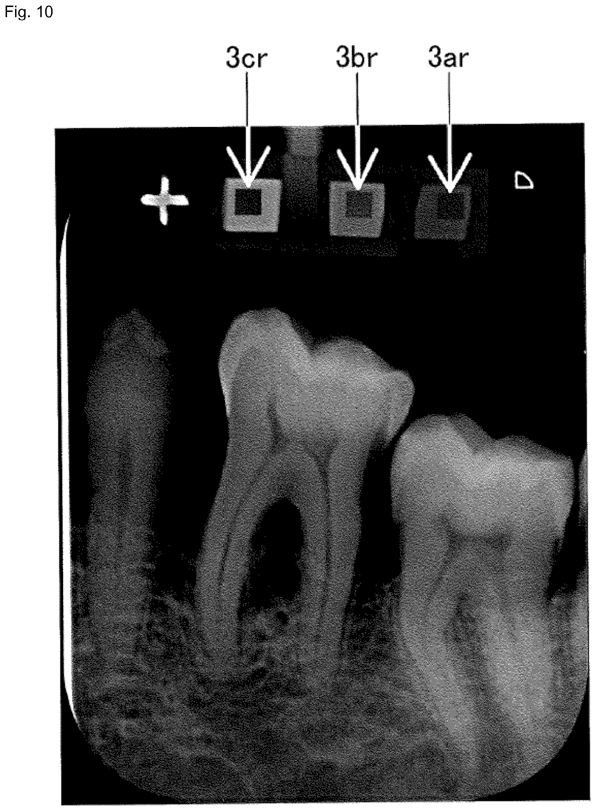

FIG. 10 is an example of an image related to a reference body region of interest setting unit of the bone density measurement system according to an embodiment of the present invention, showing a state in which the region of interest is set. As described above, the image of the reference body 3 is often photographed tilted from the horizontal direction, and a correction is necessary because there are subtle errors due to the influence of the X-ray incident angle and so on. The image of the reference body is confirmed from the relative value of the distance to the reference body sample 3a, 3b, 3c from the image of, and further, the region of interest is created with a coefficient that minimizes the variance of the concentration in the region of interest. In some cases, a method such as

Thereafter, areas of a predetermined number of pixels in the vertical and horizontal directions are set from the center coordinates of the reference body samples 3a, 3b, 3c. This is the reference body region of interest 3ar, 3br, 3cr. Here, the size of each of the reference body regions of interest 3ar, 3br, and 3cr is a square with one side of 30 pixels.

By doing this, the region of interest of the reference body can be automatically and accurately performed without human intervention, and the effect is large.

Of course, even if the region of interest of the reference body can not be automatically processed for some reason, the image shown in FIG. 9 may be presented on the screen to prompt the operator for input.

Next, the reference body density measurement unit 103 measures the image density of the reference body samples 3a, 3b, and 3c from the image of the region of interest of the reference body 3 by a known method (Step S04 in FIG. 6). Here, as a representative value of concentration, any of average value, median value and mode value can be set according to the situation.

For example, as for the concentrations of the reference bodies 3a, 3b and 3c, it is preferable to use the central value as the representative value of the concentration, so it is desirable to use an average value or median value, especially when the concentration distribution is stable. Average value is most desirable. On the other hand, in the case of a concentration distribution in which only a portion is pointed like the concentration of the marker 32, it is preferable to use the mode as it is preferable to set the pointed portion as the representative value of the concentration.

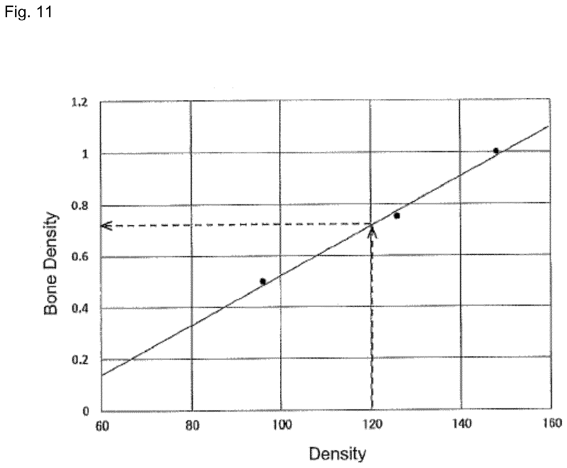

FIG. 11 is a diagram showing the correlation between the density of an image of a reference body and bone density in an embodiment of the present invention. That is, in the case of the above-mentioned example, as a result of measurement, it became as follows.

Reference body 3a concentration 96

Reference body 3b concentration 135

Reference body 3c concentration 152

Here, since the reference body 3 b and the reference body 3 c overlap with the holding portion 41 or the support portion 42 of the photographing aid 4 and the density becomes excessively high, the portions are corrected.

Reference body 3a density 96 (without correction)

Reference body 3b Concentration 135 x 0.93 = 126

Reference 3c Concentration 152 x 0.97 = 148

As a result, the relationship between concentration and bone density mentioned above is as follows.

Reference body 3a concentration 96 bone density equivalent value 0.50 g / square cm

Reference body 3b concentration 126 bone density equivalent value 0.75 g / square cm

Reference body 3c concentration 148 bone density equivalent value 1.00 g / square cm

This figure is illustrated in the figure, and a substantially linear correlation is observed. Here, the approximate expression in this case is y = 0.0095x?0.427, where the concentration is x and the bone density is y.

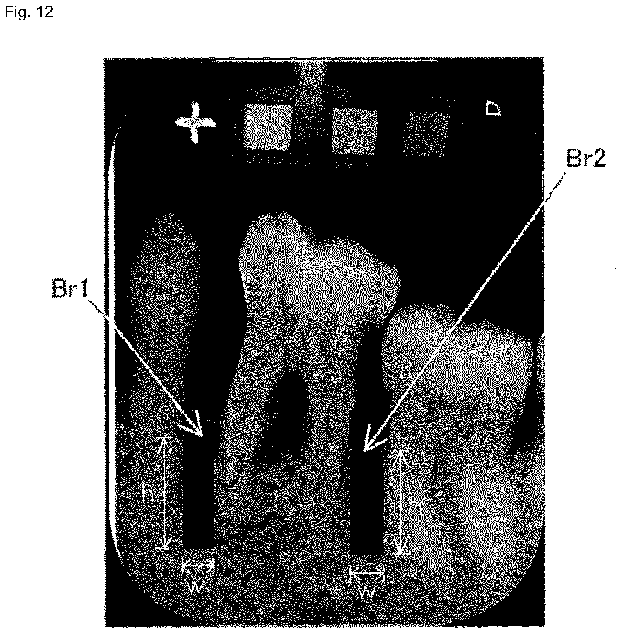

Next, the bone part region of interest setting unit 104 sets a bone portion, in particular, a region of interest Br of the alveolar bone part (step S05 in FIG. 6). FIG. 12 is an explanatory view showing a method of setting a bone region of interest in the bone densitometry device according to one embodiment of the present invention, and the width w at a position approximately equidistantly spaced on both sides of a tooth axis of a specific tooth; Two bone regions of interest Br1 and Br2 of the dimension of height h are set. The specification of the teeth and the specification of the area may be appropriately determined according to the application of bone density measurement.

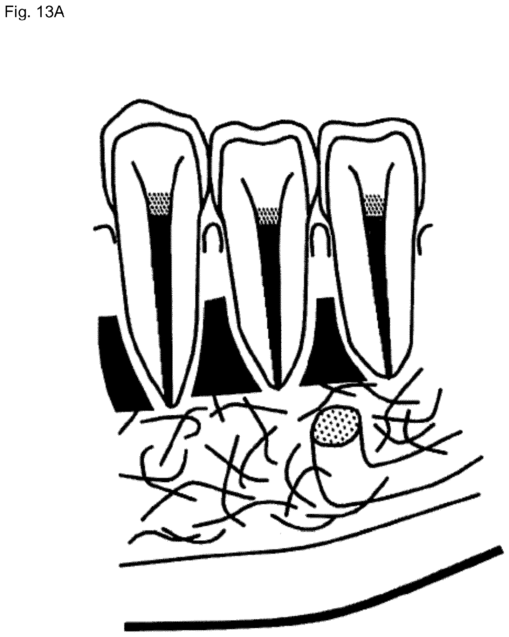

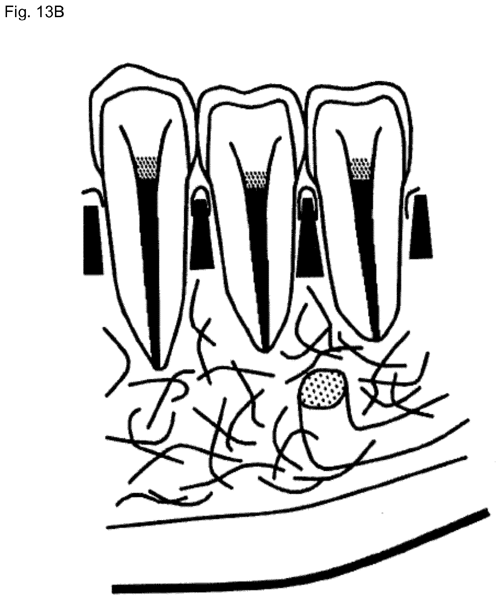

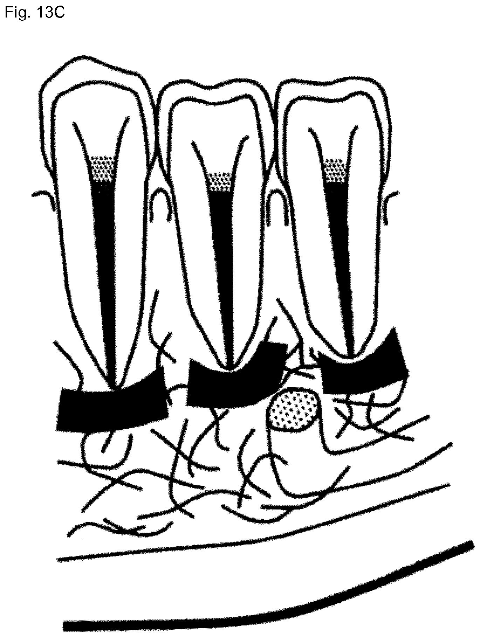

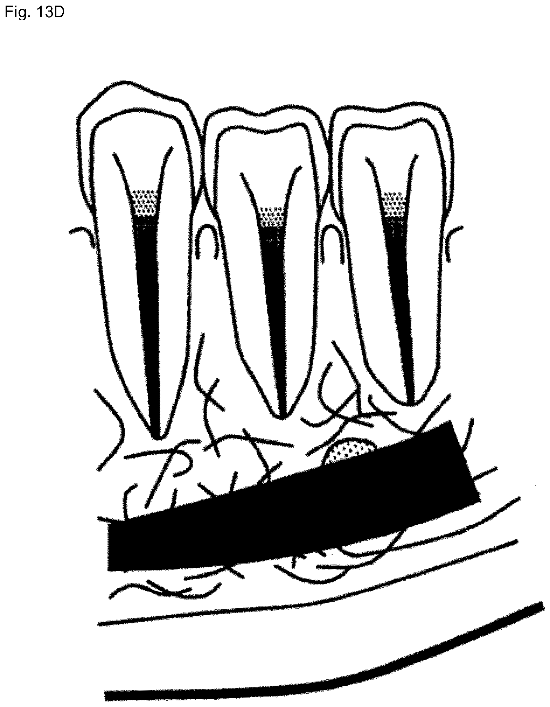

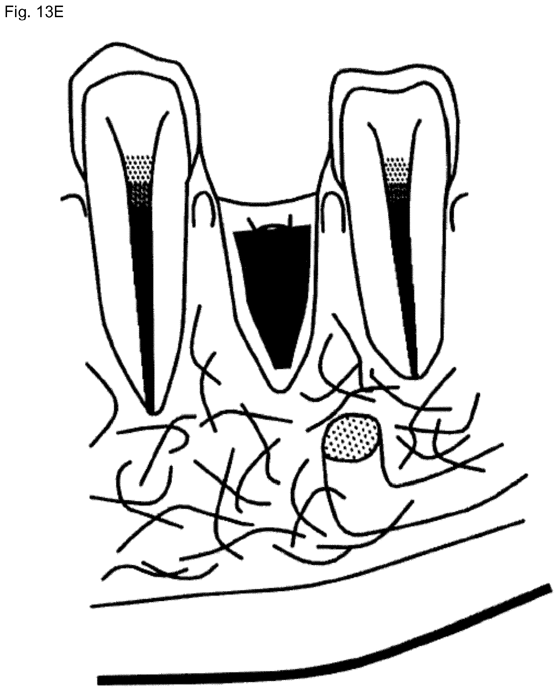

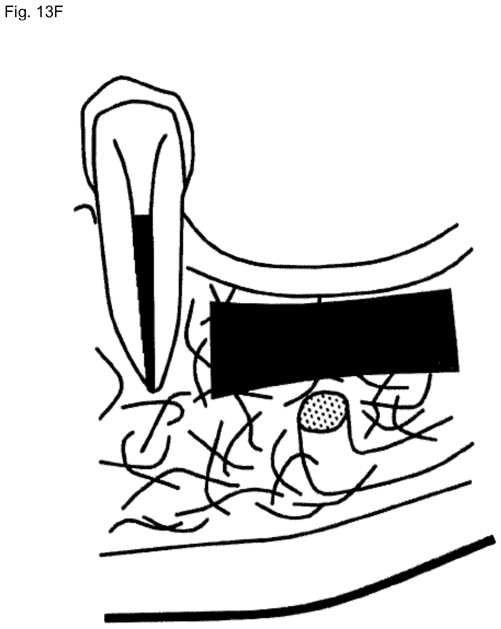

Here, the bone region of interest setting the region of interest may be elsewhere. FIGS. 13A to 13F are explanatory views showing an example of a bone region of interest in a bone density measurement device according to an embodiment of the present invention, wherein the black parts indicate the bone region of interest and the alveolar bone mentioned above Alveolar crest (FIG. 13B), apical bone (FIG. 13C), jawbone bone body (FIG. 13D), extraction pits (FIG. 13E), jaw ridges (FIG. 13E) as well as the bony bone body part It may be a part to be treated such as an implant or a technician (FIG. 13F) or the like, and the predetermined distance and size may be appropriately selected.

Here, in FIG. 13E and FIG. 13F, in particular, they are useful for evaluating the progress of treatment and planning of treatment planning for the dentist, and the others are particularly based on the quantitative evaluation of alveolar bone resorption. It is useful for diagnosis support of periodontal disease.

As for the region of interest of the bone portion, the region may not necessarily be set, and a specific portion may be designated to measure the concentration at that portion.

Next, the bone part concentration measurement unit 105 measures the concentration of the bone part, in particular, the region of interest in the alveolar bone part (step S06 in FIG. 6). For example, a representative value of the concentration is determined for the bone region of interest in the alveolar bone portion described above. The region of interest of the alveolar bone portion can be a region of interest that simultaneously includes a tooth region and a non-tooth region because the user can arbitrarily select it. In such a case, after observing the concentration distribution, the representative value is set to one of an average value, a median value, and a mode value. Here, it is assumed that, for example, the concentration is determined to be 120 as a representative value.

Although it is preferable to obtain the concentration as a representative value of the region of interest from the viewpoint of securing the accuracy, the concentration may be obtained by other methods such as the concentration of only a specific part. In some cases, it can be measured simply.

Next, the bone density calculation unit 106 calculates the bone density of the alveolar bone from the correlation between the density of the reference body and the bone density and the concentration of the region of interest in the alveolar bone (step S07 in FIG. 6). In this case, when calculated using the approximate expression of FIG. 11, the bone density at the concentration 120 is calculated to be about 0.71 g / square cm. In this way, the bone density of the alveolar bone portion can be obtained in g / square cm, which is a general-purpose unit.

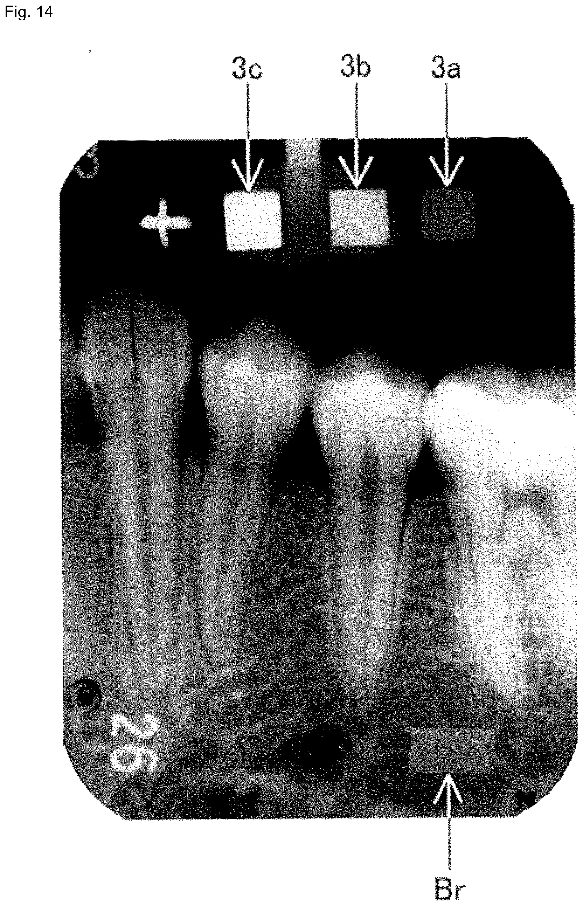

Note that the relationship between the reference body density and the bone density described above is different for each photographed image. For example, FIG. 14 is an example of a film image, not a digital image, but in this case it looks The density (pixel value) is significantly reduced, but since all of the reference body regions of interest 3a, 3b, 3c and the bone portion of interest region Br are similarly reduced in density, FIG. By creating a correlation diagram in the same manner, bone density can be calculated appropriately.

### Industrial applicability

The present application has broad industrial applicability in that it can measure bone density using dental x-ray images and can be used to support the diagnosis of osteoporosis and the like.

### [Description of the code]

1 Bone density measuring device
2 Shooting device
3 reference body
4 shooting aids
100 bone density measurement system

## Claims

1. A bone density measuring device (1), comprising:
an image display unit (101) for displaying an X-ray image relating to a photographed bone portion of a targeted tooth of a person and a plurality of reference bodies (3), the bone density values of which are given, on the same screen, the X-ray image being an image captured with an imaging aid (4) including a holding portion (41) for holding a planar imaging plate (21) capturing the X-ray image, the imaging plate being substantially orthogonal to the imaging main axis (Z) of the main body of the imaging aid (4), wherein at one end of the holding portion (41) an elongated rod-shaped support portion (42) is provided for supporting the holding portion (41) at an appropriate position when the support portion (42) is engaged by the person to be imaged and fixed so as to be directed towards the tooth root direction and further wherein a case (31) accommodating the reference bodies (3a, 3b, 3c) is set at the end portion of the support portion (42) on the holding portion (41) side so that the reference bodies (3a, 3b, 3c) can be detected by the imaging plate (21) together with the alveolar bone portion of the targeted tooth;
a reference body image density measuring unit (103) configured to measure the density of the X-ray image of the displayed plurality of reference bodies;
a bone portion image density measurement unit (105) configured to measure the density of the X-ray image of the displayed bone portion;
a bone density calculation unit (106) configured to calculate the bone density of the bone portion by comparing the X-ray image density of the bone portion with the X-ray image density of the reference bodies,
wherein
said bone density calculation unit (106) is configured to calculate bone density of the bone portion by (a) determining a relation between an X-ray image density of the plurality of reference bodies (3) and the corresponding bone density value, (b) approximating the determined relation to obtain a relation expressed by a linear regression line between the X-ray image densities of the reference bodies (3) and the corresponding bone density values, and (c) using said linear regression line as a reference to calculate the bone density of the bone portion based on the measured X-ray image density of the bone portion.

2. The bone density measuring device (1) according to claim 1, wherein the reference bodies (3) have a composition close to that of a tooth.

3. The bone density measuring device (1) according to claim 2, wherein the reference bodies (3) are **characterized in that** the hydroxyapatite or a hydroxyapatite homolog in which the OH group of the apatite is substituted with another element is mixed with carbon at different mixing ratios.

4. The bone density measuring device (1) according to claim 1, wherein the reference bodies (3) include a sample from which three levels of image density can be obtained, and the bone density calculation unit (106) calculates bone density using the three levels of image density.

5. The bone density measuring device (1) according to claim 1, wherein detection of the reference bodies (3) is started by reading a marker attached to a case containing the reference bodies (3).

6. The bone density measuring device (1) according to claim 5, further comprising a reference body region of interest setting unit that sets a region of interest of the reference bodies (3).

7. A bone density measuring system (100), comprising:
reference bodies (3) for bone density measurement;
an image device (2) for simultaneously imaging an X-ray image of a bone portion and an X-ray image of the reference bodies (3); and
a bone density measuring device (1) according to claim 1.

8. The bone density measuring system (100) according to claim 7, wherein the reference bodies have a composition close to that of a tooth.

9. The bone density measuring system (100) according to claim 7, wherein the reference bodies (3) are formed by mixing hydroxyapatite or a homolog of hydroxyapatite obtained by substituting the OH group of the apatite with another element and carbon in different mixing ratios.

10. The bone density measuring system (100) according to claim 7, wherein the reference bodies (3) comprise a sample capable of obtaining three levels of image density.

11. The bone density measuring system (100) according to claim 7, further comprising:
a case for housing the reference bodies (3); and
an auto-readable marker provided on the case.

12. The bone density measuring system (100) according to claim 7, further comprising a photographing aid device (4) configured to hold the reference bodies (3) to simultaneously photograph the reference bodies (3) and the bone portion.

13. The bone density measuring system (100) according to claim 12,
wherein the photographing aid device (4) comprises a mechanism configured to hold reference bodies on the side of the support portion (42) of the holding portion (41) so as to be detected by the image information detection medium together with the bone portion.

## Patentansprüche

1. Knochendichtemessgerät (1), umfassend:
eine Bildanzeigeeinheit (101) zum Anzeigen eines Röntgenbildes bezüglich eines fotografierten Knochenabschnitts eines Zielzahns einer Person und mehrerer Referenzkörper (3), deren Knochendichtewerte angegeben sind, auf demselben Bildschirm, wobei das Röntgenbild ein Bild ist, das mit einer Bildgebungshilfe (4) aufgenommen wurde, die einen Halteabschnitt (41) zum Halten einer planaren Bildgebungsplatte (21), die das Röntgenbild aufnimmt, umfasst, wobei die Bildgebungsplatte im Wesentlichen orthogonal zur Bildgebungshauptachse (Z) des Hauptkörpers der Bildgebungshilfe (4) angeordnet ist, wobei an einem Ende des Halteabschnitts (41) ein länglicher stabförmiger Stützabschnitt (42) vorgesehen ist für ein Stützen des Halteabschnitts (41) an einer geeigneten Position, wenn der Stützabschnitt (42) mit der abzubildenden Person in Eingriff steht und so fixiert ist, dass er in Richtung der Zahnwurzel gerichtet ist, und wobei des Weiteren ein Gehäuse (31), das die Referenzkörper (3a, 3b, 3c) beherbergt, an dem Endabschnitt des Stützabschnitts (42) auf der Seite des Halteabschnitts (41) so angebracht ist, dass die Referenzkörper (3a, 3b, 3c) von der Bildgebungsplatte (21) zusammen mit dem Alveolarknochenabschnitt des Zielzahns erfasst werden können,
eine Einheit zur Messung der Bilddichte eines Referenzkörpers (103), die konfiguriert ist, die Dichte des Röntgenbilds der angezeigten mehreren Referenzkörper zu messen,
eine Einheit zur Messung der Bilddichte eines Knochenabschnitts (105), die konfiguriert ist, die Dichte des Röntgenbilds des angezeigten Knochenabschnitts zu messen,
eine Einheit zur Berechnung der Knochendichte (106), die konfiguriert ist, die Knochendichte des Knochenabschnitts durch Vergleichen der Dichte des Röntgenbilds des Knochenabschnitts mit der Dichte des Röntgenbilds der Referenzkörper zu berechnen,
wobei
die Einheit zur Berechnung der Knochendichte (106) konfiguriert ist, die Berechnung der Knochendichte des Knochenabschnitts durch (a) Ermitteln einer Beziehung zwischen der Röntgenbilddichte der mehreren Referenzkörper (3) und dem entsprechenden Knochendichtewert, (b) Approximieren der ermittelten Beziehung, um eine durch eine lineare Regressionsgerade ausgedrückte Beziehung zwischen den Röntgenbilddichten der Referenzkörper (3) und den entsprechenden Knochendichtewerten zu erhalten, und (c) Verwenden der linearen Regressionsgeraden als Referenz zum Berechnen der Knochendichte des Knochenabschnitts auf Basis der gemessenen Röntgenbilddichte des Knochenabschnitts durchzuführen.

2. Knochendichtemessgerät (1) gemäß Anspruch 1, wobei die Referenzkörper (3) eine Zusammensetzung aufweisen, die derjenigen eines Zahns nahekommt.

3. Knochendichtemessgerät (1) gemäß Anspruch 2, wobei die Referenzkörper (3) **dadurch gekennzeichnet sind, dass** der Hydroxylapatit oder ein Hydroxylapatit-Homolog, bei dem die OH-Gruppe des Apatits durch ein anderes Element substituiert ist, mit Kohlenstoff in unterschiedlichen Mischungsverhältnissen vermischt ist.

4. Knochendichtemessgerät (1) gemäß Anspruch 1, wobei die Referenzkörper (3) eine Probe, von der drei Bilddichtestufen erhalten werden können, umfasst, und wobei die Einheit zur Berechnung der Knochendichte (106) die Knochendichte unter Verwendung der drei Bilddichtestufen berechnet.

5. Knochendichtemessgerät (1) gemäß Anspruch 1, wobei die Messung der Referenzkörper (3) durch Lesen einer Markierung gestartet wird, die an einem die Referenzkörper (3) enthaltenden Gehäuse angebracht ist.

6. Knochendichtemessgerät (1) gemäß Anspruch 5, das des Weiteren eine Einheit zum Festlegen eines interessierenden Bereichs der Referenzkörper umfasst, die einen interessierenden Bereich der Referenzkörper festlegt.

7. Knochendichte-Messsystem (100), umfassend:
Referenzkörper (3) zur Messung der Knochendichte,
eine Bildgebungsvorrichtung (2) zum gleichzeitigen Abbilden eines Röntgenbilds eines Knochenabschnitts und eines Röntgenbilds der Referenzkörper (3) und
eine Vorrichtung zur Messung der Knochendichte (1) gemäß Anspruch 1.

8. Knochendichte-Messsystem (100) gemäß Anspruch 7, wobei die Referenzkörper (3) eine Zusammensetzung aufweisen, die derjenigen eines Zahns nahekommt.

9. Knochendichte-Messsystem (100) gemäß Anspruch 7, wobei die Referenzkörper (3) durch Mischen von Hydroxylapatit oder einem Hydroxylapatit-Homolog, das durch Substituieren der OH-Gruppe des Apatits durch ein anderes Element erhalten wird, und Kohlenstoff in unterschiedlichen Mischungsverhältnissen gebildet sind.

10. Knochendichte-Messsystem (100) gemäß Anspruch 7, wobei die Referenzkörper (3) eine Probe umfassen, die in der Lage ist, drei Bilddichtestufen zu erhalten.

11. Knochendichte-Messsystem (100) gemäß Anspruch 7, des Weiteren umfassend:
ein Gehäuse zum Beherbergen der Referenzkörper (3) und
eine automatisch lesbare Markierung, die auf dem Gehäuse vorgesehen ist.

12. Knochendichte-Messsystem (100) gemäß Anspruch 7, das des Weiteren eine Vorrichtung zur Unterstützung des Fotografierens (4) umfasst, die zum Halten der Referenzkörper (3) für ein gleichzeitiges Fotografieren der Referenzkörper (3) und des Knochenabschnitt konfiguriert ist.

13. Knochendichte-Messsystem (100) gemäß Anspruch 12,
wobei die Vorrichtung zur Unterstützung des Fotografierens (4) einen Mechanismus umfasst, der konfiguriert ist, die Referenzkörper auf der Seite des Trägerabschnitts (42) des Halteabschnitts (41) so zu halten, dass sie mittels des Mediums zum Ermitteln von Bildinformationen zusammen mit dem Knochenabschnitt erfasst werden.

## Revendications

1. Dispositif de mesure de densité osseuse (1), comprenant :
une unité d'affichage d'image (101) pour afficher une image radiographique relative à une partie osseuse photographiée d'une dent cible d'une personne et d'une pluralité de corps de référence (3), dont les valeurs de densité osseuse sont données, sur le même écran, l'image radiographique étant une image capturée avec une auxiliaire d'imagerie (4) comprenant une partie de maintien (41) pour maintenir une plaque d'imagerie plane (21) capturant l'image radiographique, la plage d'imagerie étant sensiblement orthogonale à l'axe principal d'imagerie (Z) du corps principal de l'auxiliaire d'imagerie (4), dans lequel au niveau d'une extrémité de la partie de maintien (41) une partie de support (42) en forme de tige allongée est fournie pour supporter la partie de maintien (41) au niveau d'une position appropriée lorsque la partie de support (42) est engagée par la personne à radiographier et fixée de sorte à être dirigée vers la direction de racine de dent et en outre dans lequel un boîtier (31) logeant les corps de référence (3a, 3b, 3c) est défini au niveau de la partie d'extrémité de la partie de support (42) sur le côté de la partie de maintien (41) de sorte que les corps de référence (3a, 3b, 3c) peuvent être détectés par la plaque d'imagerie (21) conjointement avec la partie d'os alvéolaire de la dent cible ;
une unité de mesure de densité d'image de corps de référence (103) configurée pour mesurer la densité de l'image radiographique de la pluralité de corps de référence affichée ;
une unité de mesure de densité d'image de partie osseuse (105) configurée pour mesurer la densité de l'image radiographique de la partie osseuse affichée ;
une unité de calcul de densité osseuse (106) configurée pour calculer la densité osseuse de la partie osseuse en comparant la densité d'image radiographique de la partie osseuse à la densité d'image radiographique des corps de référence ; dans lequel
ladite unité de calcul de densité osseuse (106) est configurée pour calculer la densité osseuse de la partie osseuse par (a) détermination d'une relation entre une densité d'image radiographique de la pluralité de corps de référence (3) et la valeur de densité osseuse correspondante, (b) approximation de la relation déterminée pour obtenir une relation exprimée par une ligne de régression linéaire entre les densités d'image radiographique des corps de référence (3) et les valeurs de densité osseuse correspondantes, et (c) utilisation de ladite ligne de régression linéaire en tant que référence pour calculer la densité osseuse de la partie osseuse sur la base de la densité d'image radiographique mesurée de la partie osseuse.

2. Dispositif de mesure de densité osseuse (1) selon la revendication 1, dans lequel les corps de référence (3) ont une composition proche de celle d'une dent.

3. Dispositif de mesure de densité osseuse (1) selon la revendication 2, dans lequel les corps de référence (3) sont **caractérisés en ce que** l'hydroxyapatite ou un homologue d'hydroxyapatite dans lequel le groupe OH de l'apatite est substitué par un autre élément est mélangé avec du carbone selon différents rapports de mélange.

4. Dispositif de mesure de densité osseuse (1) selon la revendication 1, dans lequel les corps de référence (3) comprennent un échantillon à partir duquel trois niveaux de densité d'image peuvent être obtenus, et l'unité de calcul de densité osseuse (106) calcule la densité osseuse en utilisant les trois niveaux de densité d'image.

5. Dispositif de mesure de densité osseuse (1) selon la revendication 1, dans lequel la détection des corps de référence (3) est initiée par la lecture d'un marqueur fixé à un boîtier contenant les corps de référence (3).

6. Dispositif de mesure de densité osseuse (1) selon la revendication 5, comprenant en outre une unité de définition de région d'intérêt des corps de référence qui définit une région d'intérêt des corps de référence (3).

7. Système de mesure de densité osseuse (100), comprenant en outre:
des corps de référence (3) pour la mesure de densité osseuse ;
un dispositif d'image (2) pour l'imagerie simultanée d'une image radiographique d'une partie osseuse et d'une image radiographique des corps de référence (3) ;
et
un dispositif de mesure de la densité osseuse (1) selon la revendication 1.

8. Système de mesure de densité osseuse (100) selon la revendication 7, dans lequel les corps de référence (3) ont une composition proche de celle d'une dent.

9. Système de mesure de densité osseuse (100) selon la revendication 7, dans lequel les corps de référence (3) sont formés par mélange d'hydroxyapatite ou d'un homologue d'hydroxyapatite obtenu par substitution du groupe OH de l'apatite par un autre élément et de carbone selon différents rapports de mélange.

10. Système de mesure de densité osseuse (100) selon la revendication 7, dans lequel les corps de référence (3) comprennent un échantillon capable d'obtenir trois niveaux de densité d'image.

11. Système de mesure de densité osseuse (100) selon la revendication 7, comprenant en outre :
un boîtier pour loger les corps de référence (3) ; et
un marqueur lisible automatiquement fourni sur le boîtier.

12. Système de mesure de densité osseuse (100) selon la revendication 7, comprenant en outre un dispositif auxiliaire photographique (4) configuré pour maintenir les corps de référence (3) pour photographier simultanément les corps de référence (3) et la partie osseuse.

13. Système de mesure de densité osseuse (100) selon la revendication 12,
dans lequel le dispositif auxiliaire photographique (4) comprend un mécanisme configuré pour maintenir les corps de référence sur le côté de la partie de support (42) de la partie de maintien (41) de manière à être détectés par le milieu de détection d'informations d'image conjointement avec la partie osseuse.
